# EUROPEAN PATENT SPECIFICATION

(11) **EP 4 330 922 B1**
(45) Date of publication and mention of the grant of the patent: **16.09.2026**
(21) Application number: 22914349.0
(22) Date of filing: 19.12.2022
(51) Int. Cl.: G16H 30/40, G06T 11/00

(54) **SYSTEMS AND METHODS FOR IMAGE RECONSTRUCTION**
SYSTEME UND VERFAHREN ZUR BILDREKONSTRUKTION
SYSTÈMES ET PROCÉDÉS DE RECONSTRUCTION D'IMAGE

(30) Priority: 31.12.2021 CN 202111670384
(43) Date of publication of application: 06.03.2024
(73) Proprietor: Shanghai United Imaging Healthcare Co., Ltd., Shanghai 201807 (CN)
(72) Inventor: WANG, Xin, Shanghai 201807 (CN); GUO, Weiqiang, Shanghai 201807 (CN)
(74) Representative: Wang, Bo
(86) International application number: PCT/CN2022/139918
(87) International publication number: WO 2023/125095

(56) References cited:
- CN-A- 106 775 530
- CN-A- 106 775 530
- CN-A- 108 022 229
- CN-A- 110 942 490
- CN-A- 110 942 490
- CN-A- 111 084 633
- CN-A- 111 084 633
- CN-A- 114 299 187
- US-A1- 2018 047 188

## Description

### CROSS-REFERENCE TO RELATED APPLICATIONS

This application claims priority of Chinese Patent Application No. 202111670384.4, filed on December 31, 2021.

### TECHNICAL FIELD

The present disclosure generally relates to image processing, and more particularly, relates to systems and methods for image reconstruction.

### BACKGROUND

Medical imaging techniques including, e.g., computed tomography (CT), are widely used in clinical diagnosis and/or treatment. During a scan of a subject, a preview image can be reconstructed based on scan data of the subject for a user (e.g., a doctor) to view. However, the amount of CT scan data of the subject can be relatively large (e.g., several gigabytes (GB)), and the time for the CT scan data to be transmitted from a slip ring of a CT device to a reconstruction processor can be relatively long. In addition, the accuracy and efficiency of subsequent scans of the subject rely on real-time reconstruction and display of the preview image, making data transmission and subsequent image reconstruction crucial for such scenarios. Thus, it is desirable to provide a system and method for data transmission and image reconstruction, thereby improving the accuracy and/or efficiency of medical analysis and/or diagnosis.

CN 110 942 490 A discloses a CT data transmission method where a data acquisition board on the rotating part extracts imaging preview data corresponding to a preview image and transmits it to an external device via a slip ring for preview image reconstruction.

CN 111 084 633 A discloses an X-ray device having a data processing device integrated on the rotating gantry that uses scan data for image reconstruction and outputs the reconstructed image through a slip ring to a display device.

### SUMMARY

According to an aspect of the present disclosure, a system for image reconstruction may include at least one storage medium including a set of instructions, and at least one processor in communication with the at least one storage medium. When executing the set of instructions, the at least one processor may be directed to cause the system to perform a method. The method may include obtaining, by a peripheral processor, scan data of a subject from a data acquisition module of a medical device. The method may include generating, by the peripheral processor, a preview image of the subject based on the scan data. The method may include transmitting, by the peripheral processor, the preview image to a display module for display.

In some embodiments, the method may include transmitting, by the peripheral processor, the preview image to a slip ring of the medical device. The method may include transmitting, by the slip ring, the preview image to the display module for display.

In some embodiments, the method may include obtaining, by a reconstruction processor, the scan data from the slip ring of the medical device. The scan data may be transmitted from the data acquisition module to the slip ring. The method may include generating, by the reconstruction processor, a full quality image of the subject based on the scan data.

The method includes compressing, by the data acquisition module, the scan data to generate compressed scan data. The method includes obtaining, by the peripheral processor, the compressed scan data from the data acquisition module.

In some embodiments, the method may include compressing, by the peripheral processor, the scan data to generate compressed scan data. The method may include generating, by the peripheral processor, the preview image of the subject based on the compressed scan data.

In some embodiments, the method may include performing, by the peripheral processor, a preprocessing operation on the scan data to generate processed scan data. The method may include generating, by the peripheral processor, the preview image of the subject based on the processed scan data.

In some embodiments, the preprocessing operation may include at least one of an air correction processing operation, a bad channel correction processing operation, a nonlinear correction processing operation, a crosstalk correction processing operation, a calibration correction processing operation, or a convolution filter processing operation.

In some embodiments, the method may include performing, by the peripheral processor, a preprocessing operation on the scan data to generate processed scan data. The method may include transmitting, by the peripheral processor, the processed scan data to the slip ring of the medical device.

In some embodiments, the method may include transmitting, by the slip ring, the processed scan data to a reconstruction processor. The method may include generating, by the reconstruction processor, at least one image of the subject based on the processed scan data.

In some embodiments, the medical device may include a CT device. The data acquisition module may include a detection module.

The peripheral processor is an edge computing device mounted on a rotation module of a gantry of the CT device.

According to another aspect of the present disclosure, a method for image reconstruction may be implemented on a computing device including at least one processor and at least one storage device. The method may include obtaining, by a peripheral processor, scan data of a subject from a data acquisition module of a medical device. The method may include generating, by the peripheral processor, a preview image of the subject based on the scan data. The method may include transmitting, by the peripheral processor, the preview image to a display module for display.

According to another aspect of the present disclosure, a non-transitory computer readable medium may include at least one set of instructions. When executed by at least one processor of a computing device, the at least one set of instructions may cause the at least one processor to effectuate a method. The method may include obtaining, by a peripheral processor, scan data of a subject from a data acquisition module of a medical device. The method may include generating, by the peripheral processor, a preview image of the subject based on the scan data. The method may include transmitting, by the peripheral processor, the preview image to a display module for display.

According to another aspect of the present disclosure, a system for image reconstruction may include an obtaining module, a generation module, and a transmitting module. The obtaining module may be configured to obtain scan data of a subject from a data acquisition module of a medical device. The generation module may be configured to generate a preview image of the subject based on the scan data. The transmitting module may be configured to transmit the preview image to a display module for display.

According to another aspect of the present disclosure, a device may be configured to perform a method for image reconstruction. The method may include obtaining, by a peripheral processor, scan data of a subject from a data acquisition module of a medical device. The method may include generating, by the peripheral processor, a preview image of the subject based on the scan data. The method may include transmitting, by the peripheral processor, the preview image to a display module for display.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary medical system according to some embodiments of the present disclosure;
FIG. 2 is a schematic diagram illustrating exemplary hardware and/or software components of an exemplary computing device according to some embodiments of the present disclosure;
FIG. 3 is a schematic diagram illustrating exemplary hardware and/or software components of an exemplary mobile device according to some embodiments of the present disclosure;
FIG. 4A is a block diagram illustrating an exemplary peripheral processor according to some embodiments of the present disclosure;
FIG. 4B is a block diagram illustrating an exemplary reconstruction processor according to some embodiments of the present disclosure;
FIG. 5 is a flowchart illustrating an exemplary process for generating a preview image according to some embodiments of the present disclosure;
FIG. 6 is a flowchart illustrating an exemplary process for generating a full quality image according to some embodiments of the present disclosure;
FIG. 7A is a schematic diagram illustrating an exemplary process for image reconstruction in prior arts;
FIG. 7B is a schematic diagram illustrating an exemplary process for image reconstruction according to some embodiments of the present disclosure; and
FIG. 8 is a schematic diagram illustrating an exemplary medical device according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure.

The terminology used herein is to describe particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the terms "system," "unit," "module," and/or "block" used herein are one method to distinguish different components, elements, parts, sections, or assemblies of different levels in ascending order. However, the terms may be displaced by another expression if they achieve the same purpose.

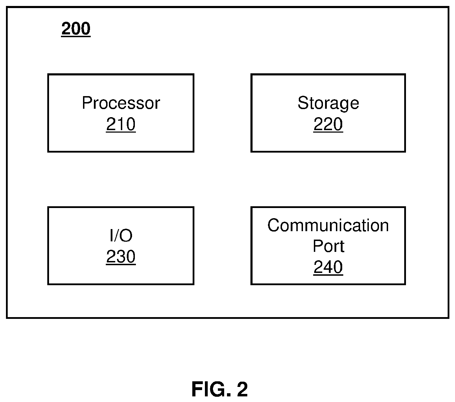

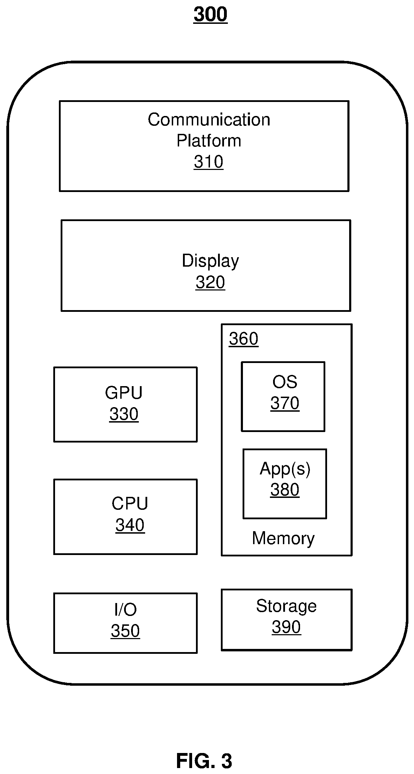

Generally, the words "module," "unit," or "block," as used herein, refer to logic embodied in hardware or firmware, or to a collection of software instructions. A module, a unit, or a block described herein may be implemented as software and/or hardware and may be stored in any type of non-transitory computer-readable medium or another storage device. In some embodiments, a software module/unit/block may be compiled and linked into an executable program. It will be appreciated that software modules can be callable from other modules/units/blocks or from themselves, and/or may be invoked in response to detected events or interrupts. Software modules/units/blocks configured for execution on computing devices (e.g., the processor 210 illustrated in FIG. 2 and/or the central processing unit (CPU) 340 illustrated FIG. 3) may be provided on a computer-readable medium, such as a compact disc, a digital video disc, a flash drive, a magnetic disc, or any other tangible medium, or as a digital download (and can be originally stored in a compressed or installable format that needs installation, decompression, or decryption prior to execution). Such software code may be stored, partially or fully, on a storage device of the executing computing device, for execution by the computing device. Software instructions may be embedded in firmware, such as an EPROM. It will be further appreciated that hardware modules/units/blocks may be included in connected logic components, such as gates and flip-flops, and/or can be included of programmable units, such as programmable gate arrays or processors. The modules/units/blocks or computing device functionality described herein may be implemented as software modules/units/blocks, but may be represented in hardware or firmware. In general, the modules/units/blocks described herein refer to logical modules/units/blocks that may be combined with other modules/units/blocks or divided into sub-modules/sub-units/sub-blocks despite their physical organization or storage. The description may apply to a system, an engine, or a portion thereof.

It will be understood that when a unit, engine, module or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

The flowcharts used in the present disclosure illustrate operations that systems implement according to some embodiments of the present disclosure. It is to be expressly understood, the operations of the flowcharts may be implemented not in order. Conversely, the operations may be implemented in inverted order, or simultaneously. Moreover, one or more other operations may be added to the flowcharts. One or more operations may be removed from the flowcharts.

An aspect of the present disclosure relates to systems and methods for image reconstruction. A peripheral processor (e.g., an edge computing device) may obtain scan data of a subject from a data acquisition module (e.g., a detection module) of a medical device (e.g., a CT device). The peripheral processor may generate a preview image of the subject based on the scan data. The peripheral processor may transmit the preview image to a slip ring of the medical device. The slip ring may transmit the preview image to a display module (e.g., a terminal device) for display. In some embodiments, a reconstruction processor may obtain the scan data from the slip ring of the medical device. The scan data may be transmitted from the data acquisition module to the slip ring. The reconstruction processor may generate a full quality image of the subject based on the scan data. The reconstruction processor may transmit the full quality image to the display module for display.

According to some embodiments of the present disclosure, the peripheral processor may generate the preview image and transmit the preview image to the display module for display, and the reconstruction processor may generate the full quality image and transmit the full quality image to the display module for display. The reconstruction of the full quality image may not be affected by the reconstruction of the preview image, which may improve the efficiency of the preview image reconstruction and the full quality image reconstruction, and may avoid the competition for processor resources between the preview image reconstruction and the full quality image reconstruction. In addition, since the data amount of the preview image (e.g., dozens of megabytes (MBs)) is less than the data amount of the scan data or preview scan data (e.g., several GBs) (e.g., the data amount of the scan data or the preview scan data may be dozens of times larger than the data amount of the preview image), the time for the preview image to be transmitted from the peripheral processor to the display module via the slip ring may be less than the time for the scan data to be transmitted from the data acquisition module to the reconstruction processor for preview image reconstruction via the slip ring, which may improve the efficiency of preview image reconstruction and ensure the real-time display of the preview image. Furthermore, the preview image may be transmitted from the peripheral processor to the display module via the slip ring, and the preview scan data (e.g., compressed scan data) may not need to be transmitted to the reconstruction processor via the slip ring, which may reduce the transmission bandwidth of the slip ring and the performance requirements for slip ring may also be reduced. Specifically, for a chest spiral scanning protocol, the amount of the raw data (e.g., full quality scan data) may be about 1 GB, and the amount of the preview scan data may be about 300 MB. The data amount of 60 preview images generated based on the preview scan data may be about 30 MB. The transmission bandwidth of the preview images may be 1/10 of the transmission bandwidth of the preview scan data. In a traditional image reconstruction process, the raw data and the preview scan data may both be transmitted via the slip ring, that is, the transmission bandwidth of the slip ring may be about 1.3 GB. According to some embodiments of the present disclosure, the raw data and the preview image may be transmitted via the slip ring and the preview scan data may not need to be transmitted via the slip ring, that is, the transmission bandwidth of the slip ring may be about 1.03 GB. Therefore, compared with the traditional image reconstruction process, 20% transmission bandwidth of the slip ring may be saved according to the image reconstruction process of the present disclosure.

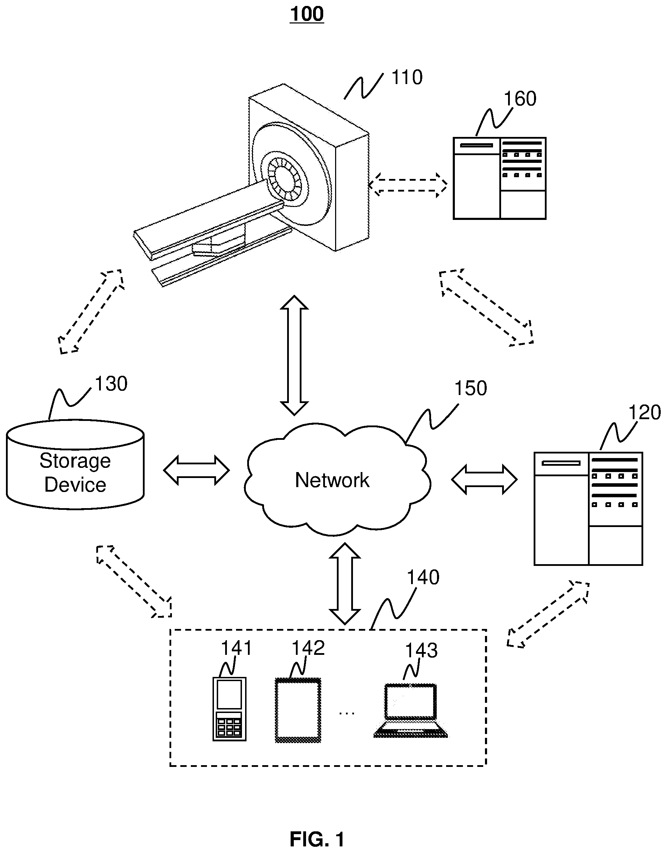

FIG. 1 is a schematic diagram illustrating an exemplary medical system according to some embodiments of the present disclosure. As illustrated in FIG. 1, the medical system 100 includes a medical device 110, a reconstruction processor 120, a storage device 130, a terminal device 140, a network 150, and a peripheral processor 160. In some embodiments, two or more components of the medical system 100 may be connected to and/or communicate with each other via a wireless connection, a wired connection, or a combination thereof. The medical system 100 may include various types of connection between its components. For example, the medical device 110 may be connected to the reconstruction processor 120 through the network 150, or connected to the reconstruction processor 120 directly as illustrated by the bidirectional dotted arrow connecting the medical device 110 and the reconstruction processor 120 in FIG. 1. As another example, the terminal device 140 may be connected to the reconstruction processor 120 through the network 150, or connected to the reconstruction processor 120 directly as illustrated by the bidirectional dotted arrow connecting the terminal device 140 and the reconstruction processor 120 in FIG. 1. As still another example, the storage device 130 may be connected to the medical device 110 through the network 150, or connected to the medical device 110 directly as illustrated by the bidirectional dotted arrow connecting the medical device 110 and the storage device 130 in FIG. 1. As still another example, the storage device 130 may be connected to the terminal device 140 through the network 150, or connected to the terminal device 140 directly as illustrated by the bidirectional dotted arrow connecting the terminal device 140 and the storage device 130 in FIG. 1.

The medical device 110 may be configured to acquire imaging data relating to a subject. The imaging data relating to a subject may include an image (e.g., an image slice), scan data (e.g., projection data), or a combination thereof. In some embodiments, the imaging data may be two-dimensional (2D) imaging data, three-dimensional (3D) imaging data, four-dimensional (4D) imaging data, or the like, or any combination thereof. The subject may be biological or non-biological. For example, the subject may include a patient, a man-made object, etc. As another example, the subject may include a specific portion, an organ, and/or tissue of the patient. Specifically, the subject may include the head, the neck, the thorax, the heart, the stomach, a blood vessel, soft tissue, a tumor, or the like, or any combination thereof. In the present disclosure, "object" and "subject" are used interchangeably.

The medical device 110 includes a computer tomography (CT) device.

In some embodiments, the medical device 110 may transmit the imaging data via the network 150 to the reconstruction processor 120, the storage device 130, the terminal device 140, and/or the peripheral processor 160. For example, the imaging data may be sent to the reconstruction processor 120 and/or the peripheral processor 160 for further processing, or may be stored in the storage device 130.

The reconstruction processor 120 may process data and/or information. The data and/or information may be obtained from the medical device 110 or retrieved from the storage device 130, the terminal device 140, the peripheral processor 160, and/or an external device (external to the medical system 100) via the network 150. The reconstruction processor 120 obtains scan data from a slip ring of a medical device (e.g., the medical device 110). As another example, the reconstruction processor 120 may generate a full quality image of a subject based on scan data. As still another example, the reconstruction processor 120 may transmit the full quality image to a display module (e.g., the terminal device 140) for display. In some embodiments, the reconstruction processor 120 may be a single server or a server group. The server group may be centralized or distributed. In some embodiments, the reconstruction processor 120 may be local or remote. For example, the reconstruction processor 120 may access information and/or data from the medical device 110, the storage device 130, the peripheral processor 160, and/or the terminal device 140 via the network 150. As another example, the reconstruction processor 120 may be directly connected to the medical device 110, the terminal device 140, the peripheral processor 160, and/or the storage device 130 to access information and/or data. In some embodiments, the reconstruction processor 120 may be implemented on a cloud platform. For example, the cloud platform may include a private cloud, a public cloud, a hybrid cloud, a community cloud, a distributed cloud, an inter-cloud, a multi-cloud, or the like, or any combination thereof. The reconstruction processor 120 is not part of the medical device 110.

The storage device 130 may store data, instructions, and/or any other information. In some embodiments, the storage device 130 may store data obtained from the medical device 110, the reconstruction processor 120, the peripheral processor 160, and/or the terminal device 140. The data may include imaging data acquired by the reconstruction processor 120, algorithms and/or models for processing the imaging data, etc. For example, the storage device 130 may store scan data obtained from a medical device (e.g., the medical device 110). As another example, the storage device 130 may store a preview image generated by the peripheral processor 160. As still another example, the storage device 130 may store a full quality image generated by the reconstruction processor 120. In some embodiments, the storage device 130 may store data and/or instructions that the reconstruction processor 120, the peripheral processor 160, and/or the terminal device 140 may execute or use to perform exemplary methods described in the present disclosure. In some embodiments, the storage device 130 may include a mass storage, a removable storage, a volatile read-and-write memory, a read-only memory (ROM), or the like, or any combination thereof. Exemplary mass storages may include a magnetic disk, an optical disk, a solid-state drive, etc. Exemplary removable storages may include a flash drive, a floppy disk, an optical disk, a memory card, a zip disk, a magnetic tape, etc. Exemplary volatile read-and-write memories may include a random-access memory (RAM). Exemplary RAM may include a dynamic RAM (DRAM), a double date rate synchronous dynamic RAM (DDR SDRAM), a static RAM (SRAM), a thyristor RAM (T-RAM), and a zero-capacitor RAM (Z-RAM), etc. Exemplary ROM may include a mask ROM (MROM), a programmable ROM (PROM), an erasable programmable ROM (EPROM), an electrically erasable programmable ROM (EEPROM), a compact disk ROM (CD-ROM), and a digital versatile disk ROM, etc. In some embodiments, the storage device 130 may be implemented on a cloud platform. Merely by way of example, the cloud platform may include a private cloud, a public cloud, a hybrid cloud, a community cloud, a distributed cloud, an inter-cloud, a multi-cloud, or the like, or any combination thereof.

In some embodiments, the storage device 130 may be connected to the network 150 to communicate with one or more other components in the medical system 100 (e.g., the reconstruction processor 120, the terminal device 140, the peripheral processor 160). One or more components in the medical system 100 may access the data or instructions stored in the storage device 130 via the network 150. In some embodiments, the storage device 130 may be integrated into the medical device 110 or the terminal device 140.

The terminal device 140 may be connected to and/or communicate with the medical device 110, the reconstruction processor 120, the peripheral processor 160, and/or the storage device 130. In some embodiments, the terminal device 140 may include a mobile device 141, a tablet computer 142, a laptop computer 143, or the like, or any combination thereof. For example, the mobile device 141 may include a mobile phone, a personal digital assistant (PDA), a gaming device, a navigation device, a point of sale (POS) device, a laptop, a tablet computer, a desktop, or the like, or any combination thereof. In some embodiments, the terminal device 140 may include an input device, an output device, etc. The input device may include alphanumeric and other keys that may be input via a keyboard, a touchscreen (for example, with haptics or tactile feedback), a speech input, an eye tracking input, a brain monitoring system, or any other comparable input mechanism. Other types of the input device may include a cursor control device, such as a mouse, a trackball, or cursor direction keys, etc. The output device may include a display, a printer, or the like, or any combination thereof.

The network 150 may include any suitable network that can facilitate the exchange of information and/or data for the medical system 100. In some embodiments, one or more components of the medical system 100 (e.g., the medical device 110, the reconstruction processor 120, the storage device 130, the terminal device 140, the peripheral processor 160) may communicate information and/or data with one or more other components of the medical system 100 via the network 150. For example, the reconstruction processor 120, the peripheral processor 160, and/or the terminal device 140 may obtain scan data from the medical device 110 via the network 150. As another example, the reconstruction processor 120, the peripheral processor 160, and/or the terminal device 140 may obtain information stored in the storage device 130 via the network 150. The network 150 may be and/or include a public network (e.g., the Internet), a private network (e.g., a local area network (LAN), a wide area network (WAN), etc.), a wired network (e.g., an Ethernet network), a wireless network (e.g., a Wi-Fi network), a cellular network (e.g., a long term evolution (LTE) network), a frame relay network, a virtual private network (VPN), a satellite network, a telephone network, routers, hubs, witches, server computers, and/or any combination thereof. For example, the network 150 may include a cable network, a wireline network, a fiber-optic network, a telecommunications network, an intranet, a wireless local area network (WLAN), a metropolitan area network (MAN), a public telephone switched network (PSTN), a Bluetooth^{™} network, a ZigBee^{™} network, a near field communication (NFC) network, or the like, or any combination thereof. In some embodiments, the network 150 may include one or more network access points. For example, the network 150 may include wired and/or wireless network access points such as base stations and/or internet exchange points through which one or more components of the medical system 100 may be connected to the network 150 to exchange data and/or information.

The peripheral processor 160 may process data and/or information. The data and/or information may be obtained from the medical device 110. For example, the peripheral processor 160 may obtain scan data of a subject from a data acquisition module (e.g., a detection module) of a medical device (e.g., the medical device 110). As another example, the peripheral processor 160 may generate a preview image of the subject based on the scan data. As still another example, the peripheral processor 160 may transmit the preview image to a slip ring of the medical device. As still another example, the peripheral processor 160 may transmit the preview image to a display module (e.g., the terminal device 140) for display.

In some embodiments, the peripheral processor 160 may be an edge computing device. For example, the peripheral processor 160 may be an advanced RISC machine (ARM)-based embedded computing device, an X86-based microprocessor, or the like, or any combination thereof. In some embodiments, the peripheral processor 160 may be mounted on one or more components of the medical device 110. For example, the peripheral processor 160 may be mounted on a rotation module of a gantry of a CT device. In some embodiments, the peripheral processor 160 is not a part of the medical device 110.

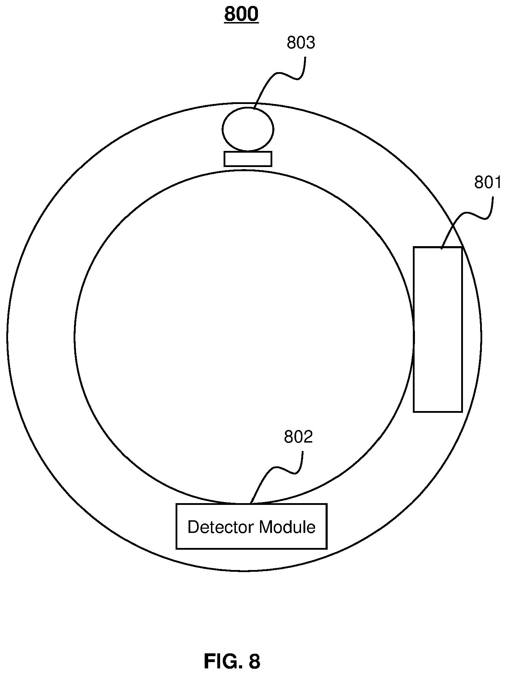

FIG. 8 is a schematic diagram illustrating an exemplary medical device according to some embodiments of the present disclosure. As illustrated in FIG. 8, a medical device 800 (e.g., a CT device) may include a rotation module 801, a detection module 802, and a tube 803. The tube 803 may emit radiation beams to a subject. The detection module 802 may detect the radiation beams and generate data associated with the projection formed by the detected radiation beams (e.g., X-rays beams) as scan data (also referred to as projection data).

In some embodiments, the size of the peripheral processor 160 may be relatively small. The peripheral processor 160 may be mounted on the rotation module 801 of a gantry of the medical device 800. In some embodiments, the peripheral processor 160 may be configured at the front end of a slip ring, and the peripheral processor 160 may process data (e.g., the scan data) prior to the slip ring of the medical device 800. For example, the peripheral processor 160 may be connected to the detection module 802 directly, and the peripheral processor 160 may obtain data (e.g., the scan data) from the detection module 802. The peripheral processor 160 may process the data (e.g., the scan data) and transmit processed data (e.g., a preview image) to the slip ring of the medical device 800.

This description is intended to be illustrative, and not to limit the scope of the present disclosure. Many alternatives, modifications, and variations will be apparent to those skilled in the art. The features, structures, methods, and other characteristics of the exemplary embodiments described herein may be combined in various ways to obtain additional and/or alternative exemplary embodiments. However, those variations and modifications do not depart the scope of the present disclosure. In some embodiments, the medical system 100 may include one or more additional components and/or one or more components of the medical system 100 described above may be omitted. Additionally or alternatively, two or more components of the medical system 100 may be integrated into a single component. A component of the medical system 100 may be implemented on two or more sub-components.

FIG. 2 is a schematic diagram illustrating exemplary hardware and/or software components of an exemplary computing device on which the reconstruction processor 120 and/or the peripheral processor 160 may be implemented according to some embodiments of the present disclosure. As illustrated in FIG. 2, a computing device 200 may include a processor 210, storage 220, an input/output (I/O) 230, and a communication port 240.

The processor 210 may execute computer instructions (e.g., program code) and perform functions of the reconstruction processor 120 and/or the peripheral processor 160 in accordance with techniques described herein. The computer instructions may include, for example, routines, programs, objects, components, data structures, procedures, modules, and functions, which perform particular functions described herein. For example, the processor 210 may process image data obtained from the medical device 110, the terminal device 140, the storage device 130, and/or any other component of the medical system 100. In some embodiments, the processor 210 may include one or more hardware processors, such as a microcontroller, a microprocessor, a reduced instruction set computer (RISC), an application specific integrated circuits (ASICs), an application-specific instruction-set processor (ASIP), a central processing unit (CPU), a graphics processing unit (GPU), a physics processing unit (PPU), a microcontroller unit, a digital signal processor (DSP), a field programmable gate array (FPGA), an advanced RISC machine (ARM), a programmable logic device (PLD), any circuit or processor capable of executing one or more functions, or the like, or any combination thereof.

Merely for illustration, only one processor is described in the computing device 200. However, it should be noted that the computing device 200 in the present disclosure may also include multiple processors. Thus operations and/or method steps that are performed by one processor as described in the present disclosure may also be jointly or separately performed by the multiple processors. For example, if in the present disclosure the processor of the computing device 200 executes both process A and process B, it should be understood that process A and process B may also be performed by two or more different processors jointly or separately in the computing device 200 (e.g., a first processor executes process A and a second processor executes process B, or the first and second processors jointly execute processes A and B).

The storage 220 may store data/information obtained from the medical device 110, the terminal device 140, the storage device 130, and/or any other component of the medical system 100. The storage 220 may be similar to the storage device 130 described in connection with FIG. 1, and the detailed descriptions are not repeated here.

The I/O 230 may input and/or output signals, data, information, etc. In some embodiments, the I/O 230 may enable a user interaction with the reconstruction processor 120 and/or the peripheral processor 160. In some embodiments, the I/O 230 may include an input device and an output device. Examples of the input device may include a keyboard, a mouse, a touchscreen, a microphone, a sound recording device, or the like, or a combination thereof. Examples of the output device may include a display device, a loudspeaker, a printer, a projector, or the like, or a combination thereof. Examples of the display device may include a liquid crystal display (LCD), a light-emitting diode (LED)-based display, a flat panel display, a curved screen, a television device, a cathode ray tube (CRT), a touchscreen, or the like, or a combination thereof.

The communication port 240 may be connected to a network (e.g., the network 150) to facilitate data communications. The communication port 240 may establish connections between the reconstruction processor 120 (or the peripheral processor 160) and the medical device 110, the terminal device 140, and/or the storage device 130. The connection may be a wired connection, a wireless connection, any other communication connection that can enable data transmission and/or reception, and/or any combination of these connections. The wired connection may include, for example, an electrical cable, an optical cable, a telephone wire, or the like, or any combination thereof. The wireless connection may include, for example, a Bluetooth^{™} link, a Wi-Fi^{™} link, a WiMax^{™} link, a WLAN link, a ZigBee link, a mobile network link (e.g., 3G, 4G, 5G), or the like, or any combination thereof. In some embodiments, the communication port 240 may be and/or include a standardized communication port, such as RS232, RS485. In some embodiments, the communication port 240 may be a specially designed communication port. For example, the communication port 240 may be designed in accordance with the digital imaging and communications in medicine (DICOM) protocol.

FIG. 3 is a schematic diagram illustrating exemplary hardware and/or software components of an exemplary mobile device according to some embodiments of the present disclosure. In some embodiments, the terminal device 140, the reconstruction processor 120, and/or the peripheral processor 160 may be implemented on a mobile device 300, respectively.

As illustrated in FIG. 3, the mobile device 300 may include a communication platform 310, a display 320, a graphics processing unit (GPU) 330, a central processing unit (CPU) 340, an I/O 350, a memory 360, and storage 390. In some embodiments, any other suitable component, including but not limited to a system bus or a controller (not shown), may also be included in the mobile device 300.

In some embodiments, the communication platform 310 may be configured to establish a connection between the mobile device 300 and other components of the medical system 100, and enable data and/or signal to be transmitted between the mobile device 300 and other components of the medical system 100. For example, the communication platform 310 may establish a wireless connection between the mobile device 300 and the medical device 110, and/or the reconstruction processor 120. The wireless connection may include, for example, a Bluetooth^{™} link, a Wi-Fi^{™} link, a WiMax^{™} link, a WLAN link, a ZigBee link, a mobile network link (e.g., 3G, 4G, 5G), or the like, or any combination thereof. The communication platform 310 may also enable the data and/or signal between the mobile device 300 and other components of the medical system 100. For example, the communication platform 310 may transmit data and/or signals inputted by a user to other components of the medical system 100. The inputted data and/or signals may include a user instruction. As another example, the communication platform 310 may receive data and/or signals transmitted from the reconstruction processor 120 and/or the peripheral processor 160. The received data and/or signals may include imaging data acquired by the medical device 110.

In some embodiments, a mobile operating system (OS) 370 (e.g., iOS^{™}, Android^{™}, Windows Phone^{™}, etc.) and one or more applications (App(s)) 380 may be loaded into the memory 360 from the storage 390 in order to be executed by the CPU 340. The applications 380 may include a browser or any other suitable mobile apps for receiving and rendering information from the reconstruction processor 120 and/or the peripheral processor 160. User interactions with the information stream may be achieved via the I/O 350 and provided to the reconstruction processor 120 (or the peripheral processor 160) and/or other components of the medical system 100 via the network 150.

To implement various modules, units, and their functionalities described in the present disclosure, computer hardware platforms may be used as the hardware platform(s) for one or more of the elements described herein. A computer with user interface elements may be used to implement a personal computer (PC) or another type of work station or terminal device, although a computer may also act as a server if appropriately programmed. It is believed that those skilled in the art are familiar with the structure, programming and general operation of such computer equipment and as a result the drawings should be self-explanatory.

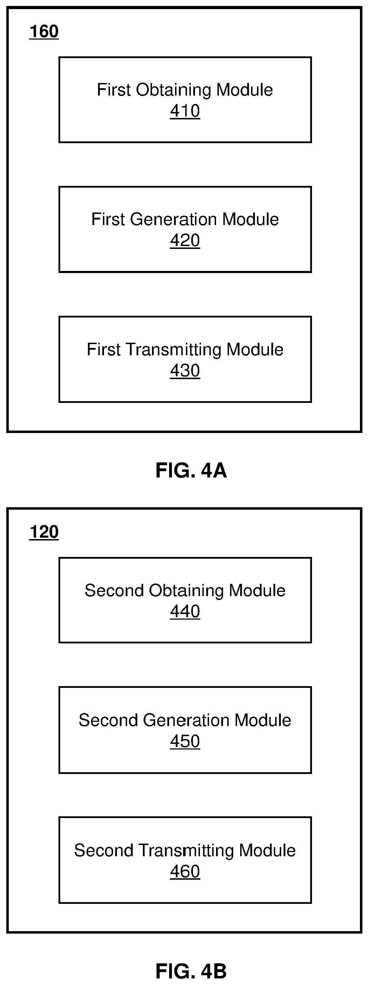

FIG. 4A is a block diagram illustrating an exemplary peripheral processor according to some embodiments of the present disclosure. In some embodiments, the peripheral processor 160 may include a first obtaining module 410, a first generation module 420, and a first transmitting module 430.

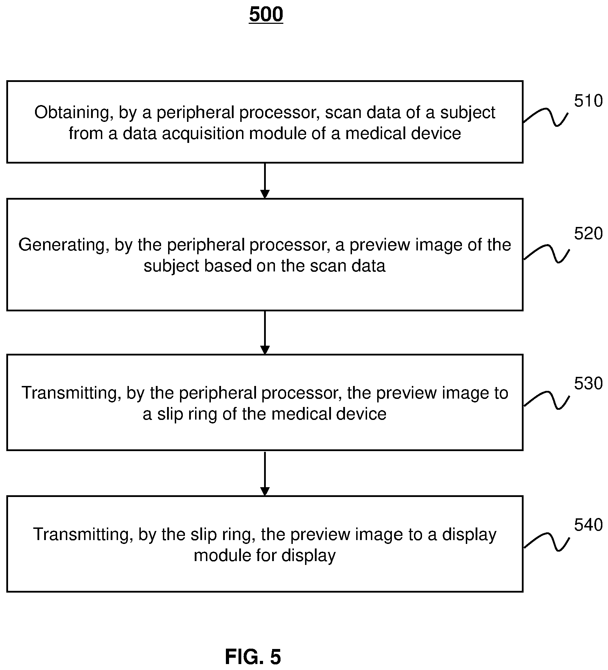

The first obtaining module 410 may be configured to obtain scan data of a subject from a data acquisition module of a medical device. In some embodiments, the scan data may include full quality scan data. For example, the scan data may include raw projection data obtained by scanning the subject using a CT device. More descriptions for obtaining the scan data may be found elsewhere in the present disclosure (e.g., operation 510 in FIG. 5, and descriptions thereof).

The first generation module 420 may be configured to generate a preview image of the subject based on scan data. For example, the first generation module 420 may compress scan data to generate compressed scan data. The first generation module 420 may generate a preview image of a subject based on the compressed scan data. As another example, the first generation module 420 may perform a preprocessing operation on scan data to generate processed scan data. The first generation module 420 may generate a preview image of a subject based on the processed scan data. More descriptions for generating the preview image may be found elsewhere in the present disclosure (e.g., operation 520 in FIG. 5, and descriptions thereof).

The first transmitting module 430 may be configured to transmit data and/or information to one or more components of the medical system 100. For example, the first transmitting module 430 may transmit a preview image to a slip ring of a medical device or a display module (e.g., the terminal device 140) for display. As another example, the first transmitting module 430 may transmit processed scan data to a slip ring of a medical device. More descriptions for transmitting the data and/or information may be found elsewhere in the present disclosure (e.g., operation 530 in FIG. 5, and descriptions thereof).

It should be noted that the above description of the peripheral processor 160 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more modules may be combined into a single module. For example, the first obtaining module 410 and the first generation module 420 may be combined into a single module.

FIG. 4B is a block diagram illustrating an exemplary reconstruction processor according to some embodiments of the present disclosure. In some embodiments, the reconstruction processor 120 may include a second obtaining module 440, a second generation module 450, and a second transmitting module 460.

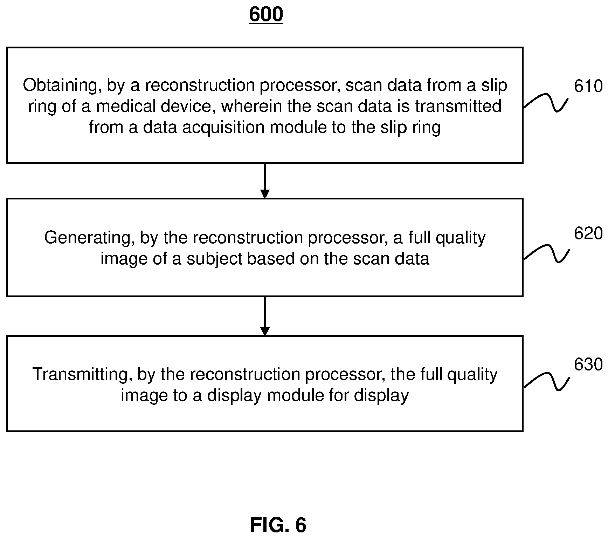

The second obtaining module 440 may be configured to obtain scan data from a slip ring of a medical device. In some embodiments, the scan data may include full quality scan data. For example, the scan data may include raw projection data obtained by scanning the subject using a CT device. More descriptions for obtaining scan data may be found elsewhere in the present disclosure (e.g., operation 610 in FIG. 6, and descriptions thereof).

The second generation module 450 may be configured to generate a full quality image of a subject based on scan data. In some embodiments, the second generation module 450 may generate a full quality image based on scan data according to one or more reconstruction algorithms as described elsewhere in the present disclosure. More descriptions for generating the preview image may be found elsewhere in the present disclosure (e.g., operation 620 in FIG. 6, and descriptions thereof).

The second transmitting module 460 may be configured to transmit data and/or information to one or more components of the medical system 100. For example, the second transmitting module 460 may transmit a full quality image to a display module for display. More descriptions for transmitting the data and/or information may be found elsewhere in the present disclosure (e.g., operation 630 in FIG. 6, and descriptions thereof).

It should be noted that the above description of the reconstruction processor 120 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more modules may be combined into a single module. For example, the second obtaining module 440 and the second generation module 450 may be combined into a single module.

FIG. 5 is a flowchart illustrating an exemplary process for generating a preview image according to some embodiments of the present disclosure. In some embodiments, process 500 may be executed by the medical system 100. For example, the process 500 may be implemented as a set of instructions (e.g., an application) stored in a storage device (e.g., the storage device 130, the storage 220, and/or the storage 390). In some embodiments, the peripheral processor 160 (e.g., the processor 210 of the computing device 200, the CPU 340 of the mobile device 300, and/or one or more modules illustrated in FIG. 4A) may execute the set of instructions and may accordingly be directed to perform the process 500. The operations of the illustrated process presented below are intended to be illustrative. In some embodiments, the process 500 may be accomplished with one or more additional operations not described and/or without one or more of the operations discussed. Additionally, the order of the operations of process 500 illustrated in FIG. 5 and described below is not intended to be limiting.

In 510, the peripheral processor 160 (e.g., the first obtaining module 410) may obtain scan data of a subject from a data acquisition module of a medical device.

In some embodiments, the scan data may include full quality scan data. As used herein, full quality scan data of a subject refers to raw data obtained by scanning the subject using a medical device (e.g., the medical device 110). For example, the scan data may include raw projection data obtained by scanning the subject using a CT device. In some embodiments, the subject may include a biological subject and/or a non-biological subject. For example, the subject may include a specific portion of a body, such as the head, the thorax, the abdomen, or the like, or any combination thereof. As another example, the subject may be a man-made composition of organic and/or inorganic matters that are with or without life.

In some embodiments, the peripheral processor 160 may obtain the scan data from the data acquisition module of the medical device. For example, the medical device may be a CT device. The data acquisition module may include a detection module. For example, the CT device may include a radiation source, the detection module, and a gantry. The radiation source and the detection module may be mounted on the gantry. The radiation source may emit radiation beams to the subject. The detection module may detect the radiation beams and generate data associated with the projection formed by the detected radiation beams (e.g., X-rays beams) as the scan data (also referred to as projection data). In some embodiments, the detection module may include one or more detector units. The detector unit(s) may include a scintillation detector (e.g., a cesium iodide detector, a gadolinium oxysulfide detector), a gas detector, etc.

The peripheral processor 160 includes an edge computing device. For example, the peripheral processor 160 may be an ARM-based embedded computing device, an X86-based microprocessor, or the like, or any combination thereof. In some embodiments, the peripheral processor 160 may be mounted on one or more components of the medical device 110. For example, the peripheral processor 160 may be mounted on a rotation module of a gantry of a CT device.

In 520, the peripheral processor 160 (e.g., the first generation module 420) may generate a preview image of the subject based on the scan data.

In some embodiments, the preview image may be used to determine current scan information (e.g., a scan range, a scan position) of the subject during the scan of the subject. In some embodiments, the quality (e.g., the resolution) requirements for the preview image may be relatively low. The requirements for real-time display of the preview image may be relatively high. For example, the preview image may need to be generated and displayed within one second or hundreds of milliseconds after the subject is scanned by the medical device.

In some embodiments, the preview image may include a CT image, a PET image, an MRI image, or the like. In some embodiments, the preview image may include a two-dimensional (2D) image, a three-dimensional (3D) image, a four-dimensional (4D) image, or the like.

In some embodiments, the peripheral processor 160 may generate the preview image based on the scan data according to one or more reconstruction algorithms. Exemplary reconstruction algorithms may include an analytic reconstruction algorithm, an iterative reconstruction algorithm, a Fourier-based reconstruction algorithm, or the like, or any combination thereof. Exemplary analytic reconstruction algorithms may include a filter back projection (FBP) algorithm, a back-projection filter (BFP) algorithm, or the like, or any combination thereof. Exemplary iterative reconstruction algorithms may include a maximum likelihood expectation maximization (ML-EM), an ordered subset expectation maximization (OSEM), a row-action maximum likelihood algorithm (RAMLA), a dynamic row-action maximum likelihood algorithm (DRAMA), an ordered subset conjugate gradiental (OSCG) algorithm, an algebraic reconstruction technique (ART), or the like, or any combination thereof. Exemplary Fourier-based reconstruction algorithm may include a classical direct Fourier algorithm, a non-uniform fast Fourier transform (NUFFT) algorithm, or the like, or any combination thereof.

The peripheral processor 160 obtains the scan data (e.g., the full quality scan data) from the data acquisition module. The peripheral processor 160 may compress the scan data to generate compressed scan data (also referred to as preview scan data). For example, the reconstruction processor 120 may compress the scan data according to a compression ratio. The compression ratio may be manually set by a user (e.g., a doctor) of the medical system 100, or determined by one or more components of the medical device 110 according to different situations. For example, the compression ratio may be 1/2, 1/4, 1/8, or the like. That is, after the scan data is compressed according to a compression ratio of 1/4, the amount of compressed scan data may be 1/4 of the amount of scan data. Further, the peripheral processor 160 may generate the preview image of the subject based on the compressed scan data.

The data acquisition module compresses the scan data to generate the compressed scan data. The peripheral processor 160 obtains the compressed scan data from the data acquisition module. The peripheral processor 160 may generate the preview image of the subject based on the compressed scan data.

According to some embodiments of the present disclosure, the amount of the compressed scan data may be smaller than the amount of the scan data, and the time for the compressed scan data to be transmitted from the data acquisition module to the peripheral processor 160 may be less than the time for the scan data to be transmitted from the data acquisition module to the peripheral processor 160, which may improve the efficiency of preview image reconstruction.

In some embodiments, the peripheral processor 160 may perform a preprocessing operation on the scan data (or the compressed scan data) to generate processed scan data. Further, the peripheral processor 160 may generate the preview image of the subject based on the processed scan data. In some embodiments, the preprocessing operation may include an air correction processing operation, a bad channel correction processing operation, a nonlinear correction processing operation, a crosstalk correction processing operation, a calibration correction processing operation, a convolution filter processing operation, or the like, or any combination thereof.

The purpose of the air correction may be to reduce the effects of response inconsistencies of channels of the detection module of the medical device, and to reduce the effects of non-uniform dose distribution of a radiation source of the medical device. In the air correction processing operation, an air correction table may be generated based on collected air data, and the scan data may be processed based on the air correction table.

The purpose of the bad channel correction may be to correct raw data corresponding to a bad channel marked by a medical system (e.g., the medical system 100). During the scan data acquisition process, an abnormality may occur in one or more channels of the detection module, resulting in errors in collected data signals. The one or more bad channels may be marked, and the corresponding raw data may be corrected. For example, the raw data corresponding to the bad channel may be determined by performing a linear interpolation operation on raw data corresponding to normal channels adjacent to the bad channel.

The purpose of the nonlinear correction may be to correct high-frequency spectral differences between channels of the detection module. Since X-rays emitted by the radiation source have multiple frequencies, different channels of the detection module may have different spectral responses, and the nonlinear response of the channels of the detection module may lead to image artifacts.

Due to X-ray scattering, light leakage, and electronic signal leakage, a signal crosstalk may exist between CT detector units. The signal crosstalk may lead to distortion of data collected by the detection module, which may lead to ring artifacts in a reconstructed image. In some embodiments, a crosstalk coefficient of each channel of the detection module may be determined using a phantom, and the crosstalk correction processing operation may be performed on a specific channel based on crosstalk coefficients of one or more channels other than the specific channel (e.g., channels adjacent to the specific channel).

The purpose of the calibration correction may be to calibrate scan data to obtain correct CT numbers. As used herein, a CT number refers to a calculated value reflecting the X-ray attenuation coefficient in an image voxel, generally expressed in Hounsfield units (HU). As used herein, Hounsfield unit (HU) refers to a dimensionless unit used in computed tomography (CT) scanning to express CT numbers in a standardized and convenient form. The CT Hounsfield scale may be calibrated such that the HU value for water is 0 HU and that for air is -1024 HU. In some embodiments, a calibration table may be generated by scanning an air phantom and a water phantom, and the calibration correction processing operation may be performed based on the calibration table.

According to some embodiments of the present disclosure, the processed scan data may be generated by performing the preprocessing operation on the scan data (or the compressed scan data), and the preview image may be generated based on the processed scan data, which may improve the image quality of the preview image.

In some embodiments, the peripheral processor 160 may perform the convolution filter processing operation on the scan data (or the compressed scan data) to generate processed scan data. Further, the peripheral processor 160 may generate the preview image by performing a back projection processing operation (e.g., a filtered back projection) on the processed scan data. Specifically, before the back projection processing operation, the scan data corresponding to each acquisition angle may be filtered to compensate for high-frequency components of the scan data and reduce the projection center density, thereby eliminating the edge unsharp effect caused by the back-projection. The scan data may then be back-projected and superimposed to obtain the preview image. By performing the convolution filter processing operation on the scan data, high-frequency information in the preview image may be increased, low-frequency information in the preview image may be suppressed, which may improve the image quality of the preview image.

In some embodiments, the peripheral processor 160 may perform a plurality of preprocessing operations on the scan data (or the compressed scan data). For example, the peripheral processor 160 may perform a correction processing operation (e.g., the air correction processing operation, the bad channel correction processing operation, the nonlinear correction processing operation, the crosstalk correction processing operation, the calibration correction processing operation) on the scan data (or the compressed scan data) to generate first processed scan data. The peripheral processor 160 may perform the convolution filter processing operation on the first processed scan data to generate second processed scan data. Further, the peripheral processor 160 may generate the preview image by performing a back projection processing operation on the second processed scan data.

In some embodiments, in the preview image reconstruction, an image post-processing operation (e.g., an artifact removal operation) may not need to be performed on the preview image, and the calculation amount of the preview image reconstruction may be relatively small. Therefore, the reconstruction of the preview image may be implemented on a small computing device (i.e., the peripheral processor 160).

In 530, the peripheral processor 160 (e.g., the first transmitting module 430) may transmit the preview image to a slip ring of the medical device.

The slip ring may be an electromechanical device that allows the transmission of power and electrical signals from a stationary to a rotating structure. For example, the slip ring of the CT device may enable power and data to be transferred without physical cables connecting the stationary and rotating portions of the CT gantry. In some embodiments, the peripheral processor 160 may transmit the preview image to the slip ring of the medical device directly or via the network 150.

In some embodiments, the peripheral processor 160 may perform the preprocessing operation on the scan data to generate processed scan data. The peripheral processor 160 may transmit the processed scan data to the slip ring of the medical device. The slip ring may transmit the processed scan data to the reconstruction processor 120. The reconstruction processor 120 may generate at least one image of the subject based on the processed scan data. For example, the reconstruction processor 120 may generate a plurality of images of the subject according to different reconstruction algorithms.

In 540, the slip ring may transmit the preview image to a display module for display.

In some embodiments, the slip ring may transmit the preview image to a display of a terminal device (e.g., the terminal device 140) for display, so as to guide a user (e.g., a doctor, a nurse, a technician, an operator of the medical device) to control the medical device to scan the subject.

In some embodiments, the peripheral processor 160 may transmit the preview image to an RF module and/or a wireless module (e.g., a WIFI module) of the medical system 100. The RF module and/or the wireless module (e.g., the WIFI module) may transmit the preview image to the display module for display. In some embodiments, the peripheral processor 160 may transmit the preview image to the display module for display directly.

It should be noted that the above description regarding the process 500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure.

FIG. 6 is a flowchart illustrating an exemplary process for generating a full quality image according to some embodiments of the present disclosure. In some embodiments, process 600 may be executed by the medical system 100. For example, the process 600 may be implemented as a set of instructions (e.g., an application) stored in a storage device (e.g., the storage device 130, the storage 220, and/or the storage 390). In some embodiments, the reconstruction processor 120 (e.g., the processor 210 of the computing device 200, the CPU 340 of the mobile device 300, and/or one or more modules illustrated in FIG. 4B) may execute the set of instructions and may accordingly be directed to perform the process 600. The operations of the illustrated process presented below are intended to be illustrative. In some embodiments, the process 600 may be accomplished with one or more additional operations not described and/or without one or more of the operations discussed. Additionally, the order of the operations of process 600 illustrated in FIG. 6 and described below is not intended to be limiting.

In 610, the reconstruction processor 120 (e.g., the second obtaining module 440) may obtain scan data from a slip ring of a medical device. The scan data may be transmitted from a data acquisition module to the slip ring.

In some embodiments, the data acquisition module (e.g., a detection module) of the medical device (e.g., a CT device) may obtain scan data by scanning the subject. The data acquisition module may transmit the scan data to the slip ring of the medical device. The reconstruction processor 120 may obtain the scan data from the slip ring.

In 620, the reconstruction processor 120 (e.g., the second generation module 450) may generate a full quality image of a subject based on the scan data.

As used herein, a full quality image refers to an image generated based on full quality scan data. In some embodiments, the full quality image may be used for disease diagnosis. In some embodiments, the quality (e.g., the resolution) requirements for the full quality image may be relatively high. The requirements for real-time display of the full quality image may be relatively low.

In some embodiments, the reconstruction processor 120 may generate the full quality image based on the scan data according to one or more reconstruction algorithms as described elsewhere in the present disclosure.

In 630, the reconstruction processor 120 (e.g., the second transmitting module 460) may transmit the full quality image to a display module for display.

In some embodiments, the reconstruction processor 120 may transmit the full quality image to a display of a terminal device (e.g., the terminal device 140) for display, so as to guide a user (e.g., a doctor, a nurse, a technician, an operator of the medical device) to perform the disease diagnosis on the subject. In some embodiments, the display module for display the full quality image may be the same as or different from the display module for display the preview image.

It should be noted that the above description regarding the process 500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure.

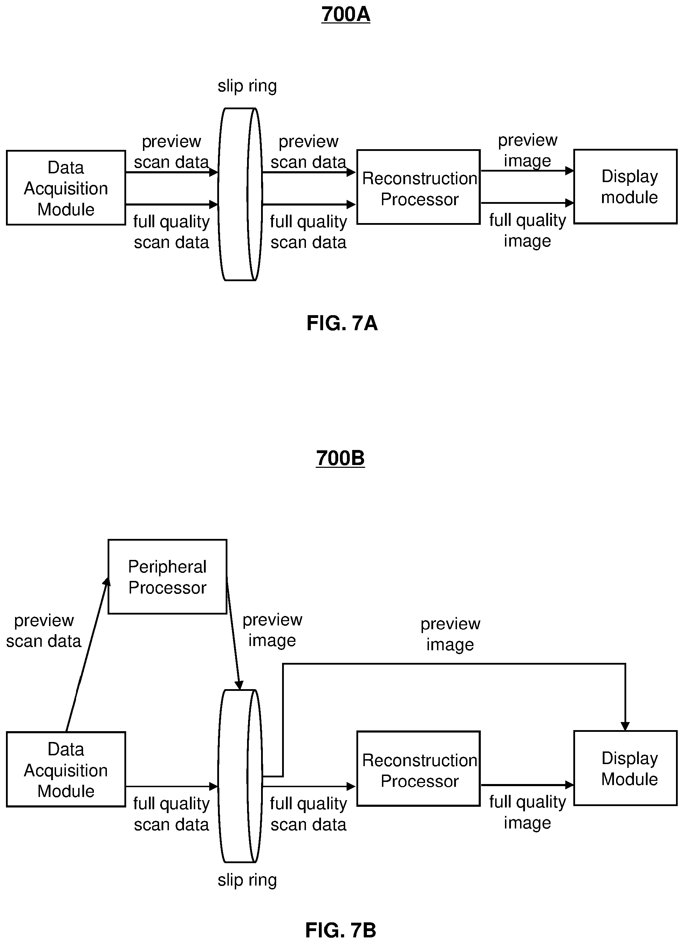

FIG. 7A is a schematic diagram illustrating an exemplary process for image reconstruction in prior arts.

As illustrated in FIG. 7A, an image reconstruction process 700A may include a preview image reconstruction and a full quality image reconstruction. In some embodiments, in the preview image reconstruction, a data acquisition module (e.g., a detector module) of a medical device may compress full quality scan data to generate preview scan data. The data acquisition module may transmit the preview scan data to a reconstruction processor via a slip ring of the medical device. The reconstruction processor may generate a preview image based on the preview scan data. The reconstruction processor may transmit the preview image to a display module for display. In the full quality image reconstruction, the data acquisition module may transmit the full quality scan data to the reconstruction processor via the slip ring of the medical device. The reconstruction processor may generate a full quality image based on the full quality scan data. The reconstruction processor may transmit the full quality image to the display module for display. That is, in prior arts, the data acquisition module may transmit the preview scan data and the full quality scan data to the reconstruction processor via the slip ring of the medical device, and the reconstruction processor may generate the preview image and the full quality image based on the preview scan data and the full quality scan data, respectively. In some embodiments, due to the amount of full quality scan data and the preview scan data is usually large (e.g., several gigabytes), it may take a long time for the preview scan data to be transmitted to the reconstruction processor via the slip ring, which may make it difficult to achieve a real-time reconstruction and display of the preview image.

FIG. 7B is a schematic diagram illustrating an exemplary process for image reconstruction according to some embodiments of the present disclosure.

As illustrated in FIG. 7B, an image reconstruction process 700B may include a preview image reconstruction and a full quality image reconstruction. In the preview image reconstruction, a peripheral processor may obtain preview scan data of a subject from a data acquisition module (e.g., a detector module) of a medical device. The peripheral processor may generate a preview image of the subject based on the preview scan data. The peripheral processor may transmit the preview image to a slip ring of the medical device. The slip ring may transmit the preview image to a display module for display. In the full quality image reconstruction, the data acquisition module may transmit the full quality scan data to the reconstruction processor via the slip ring of the medical device. The reconstruction processor may generate a full quality image based on the full quality scan data. The reconstruction processor may transmit the full quality image to the display module for display.

In a traditional image reconstruction process as illustrated in FIG. 7A, the reconstruction processor may generate the preview image and the full quality image, which may lead to a competition for reconstruction processor resources between the preview image reconstruction and the full quality image reconstruction. According to some embodiments of the present disclosure as illustrated in FIG. 7B, the preview image may be generated by the peripheral processor, and the full quality image may be generated by the reconstruction processor. The reconstruction of the full quality image may not be affected by the reconstruction of the preview image, which may improve the efficiency of preview image reconstruction and full quality image reconstruction. In addition, since the data amount of the preview image is much less than the data amount of the preview scan data (e.g., the data amount of the preview image may be dozens of megabytes and the data amount of the preview scan data may be several gigabytes), the preview image may be transmitted from the peripheral processor to the display module via the slip ring, and the preview scan data may no longer need to be transmitted to the reconstruction processor via the slip ring, which may reduce the transmission bandwidth of the slip ring, and improve the transmission efficiency of the preview image.

It should be noted that the above description regarding the process 500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. For example, in FIG. 7B, the peripheral processor may directly transmit the preview image to the display module for display. As another example, in FIG. 7B, the peripheral processor may obtain the full quality scan data from the data acquisition module. The peripheral processor may compress the full quality scan data to generate the preview scan data.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction performing system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2103, Perl, COBOL 2102, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A system for image reconstruction, comprising:
at least one storage device including a set of instructions; and
at least one processor configured to communicate with the at least one storage device, wherein when executing the set of instructions, the at least one processor is configured to direct the system to perform operations including:
obtaining, by a peripheral processor, scan data of a subject from a data acquisition module of a medical device, wherein the obtaining, by the peripheral processor, scan data of the subject from the data acquisition module of the medical device comprises:
compressing, by the data acquisition module, the scan data to generate compressed scan data; and
obtaining, by the peripheral processor, the compressed scan data from the data acquisition module;
generating, by the peripheral processor, a preview image of the subject based on the compressed scan data;
transmitting, by the peripheral processor, the preview image to a display module for display, wherein the transmitting, by the peripheral processor, the preview image to the display module for display comprises:
transmitting, by the peripheral processor, the preview image to a slip ring of the medical device; and
transmitting, by the slip ring, the preview image to the display module for display;
performing, by the peripheral processor, a preprocessing operation on the scan data to generate processed scan data;
transmitting, by the peripheral processor, the processed scan data to the slip ring of the medical device, wherein the medical device includes a computed tomography (CT) device, and the peripheral processor is an edge computing device mounted on a rotation module of a gantry of the CT device;
obtaining, by a reconstruction processor, the processed scan data from the slip ring of the medical device, wherein the reconstruction processor is not part of the medical device;
generating, by the reconstruction processor, a full quality image of the subject based on the processed scan data; and
transmitting, by the reconstruction processor, the full quality image to the display module for display.

2. The system of claim 1, wherein the generating, by the peripheral processor, a preview image of the subject based on the scan data comprises:
generating, by the peripheral processor, the preview image of the subject based on the processed scan data.

3. The system of claim 2, wherein the preprocessing operation includes at least one of an air correction processing operation, a bad channel correction processing operation, a nonlinear correction processing operation, a crosstalk correction processing operation, a calibration correction processing operation, or a convolution filter processing operation.

4. The system of claim 1, wherein the at least one processor is further configured to direct the system to perform operations including:
transmitting, by the slip ring, the processed scan data to the reconstruction processor; and
generating, by the reconstruction processor, at least one image of the subject based on the processed scan data.

5. The system of any one of claims 1-4, wherein the data acquisition module includes a detection module.

6. A method for image reconstruction, which is implemented on a computing device including at least one processor and at least one storage device, the method comprising:
obtaining, by a peripheral processor, scan data of a subject from a data acquisition module of a medical device, wherein the obtaining, by the peripheral processor, scan data of the subject from the data acquisition module of the medical device comprises:
compressing, by the data acquisition module, the scan data to generate compressed scan data; and
obtaining, by the peripheral processor, the compressed scan data from the data acquisition module;
generating, by the peripheral processor, a preview image of the subject based on the compressed scan data;
transmitting, by the peripheral processor, the preview image to a display module for display, wherein the transmitting, by the peripheral processor, the preview image to the display module for display comprises:
transmitting, by the peripheral processor, the preview image to the slip ring of the medical device; and
transmitting, by the slip ring, the preview image to the display module for display;
performing, by the peripheral processor, a preprocessing operation on the scan data to generate processed scan data;
transmitting, by the peripheral processor, the processed scan data to the slip ring of the medical device, wherein the medical device includes a computed tomography (CT) device, and the peripheral processor is an edge computing device mounted on a rotation module of a gantry of the CT device;
obtaining, by a reconstruction processor, the processed scan data from a slip ring of the medical device, wherein the reconstruction processor is not part of the medical device;
generating, by the reconstruction processor, a full quality image of the subject based on the processed scan data; and
transmitting, by the reconstruction processor , the full quality image to the display module for display.

7. A non-transitory computer readable medium, comprising executable instructions that, when executed by at least one processor, direct the at least one processor to perform a method for image reconstruction, the method comprising:
obtaining, by a peripheral processor, scan data of a subject from a data acquisition module of a medical device, wherein the obtaining, by the peripheral processor, scan data of the subject from the data acquisition module of the medical device comprises:
compressing, by the data acquisition module, the scan data to generate compressed scan data; and
obtaining, by the peripheral processor, the compressed scan data from the data acquisition module;
generating, by the peripheral processor, a preview image of the subject based on the compressed scan data;
transmitting, by the peripheral processor, the preview image to a display module for display, wherein the transmitting, by the peripheral processor, the preview image to the display module for display comprises:
transmitting, by the peripheral processor, the preview image to the slip ring of the medical device; and
transmitting, by the slip ring, the preview image to the display module for display;
performing, by the peripheral processor, a preprocessing operation on the scan data to generate processed scan data;
transmitting, by the peripheral processor, the processed scan data to the slip ring of the medical device, wherein the medical device includes a computed tomography (CT) device, and the peripheral processor is an edge computing device mounted on a rotation module of a gantry of the CT device;
obtaining, by a reconstruction processor, the processed scan data from a slip ring of the medical device, wherein the reconstruction processor is not part of the medical device;
generating, by the reconstruction processor, a full quality image of the subject based on the processed scan data; and
transmitting, by the reconstruction processor , the full quality image to the display module for display.

## Patentansprüche

1. System zur Bildrekonstruktion, umfassend:
mindestens eine Datenspeichervorrichtung, die einen Satz von Anweisungen beinhaltet; und
mindestens einen Prozessor, der dazu konfiguriert ist, mit der mindestens einen Datenspeichervorrichtung zu kommunizieren, wobei, wenn der Satz von Anweisungen ausgeführt wird, der mindestens eine Prozessor dazu konfiguriert ist, das System anzuweisen, Operationen durchzuführen, die Folgendes beinhalten:
Erhalten, durch einen Peripherieprozessor, von Abtastdaten eines Subjekts von einem Datenerfassungsmodul einer medizinischen Vorrichtung, wobei das Erhalten, durch den Peripherieprozessor, von Abtastdaten des Subjekts von dem Datenerfassungsmodul der medizinischen Vorrichtung Folgendes umfasst:
Komprimieren, durch das Datenerfassungsmodul, der Abtastdaten, um komprimierte Abtastdaten zu erzeugen; und
Erhalten, durch den Peripherieprozessor, der komprimierten Abtastdaten von dem Datenerfassungsmodul;
Erzeugen, durch den Peripherieprozessor, eines Vorschaubilds des Subjekts, basierend auf den komprimierten Abtastdaten;
Übertragen, durch den Peripherieprozessor, des Vorschaubilds an ein Anzeigemodul zur Anzeige, wobei das Übertragen, durch den Peripherieprozessor, des Vorschaubilds an das Anzeigemodul zur Anzeige, Folgendes umfasst:
Übertragen, durch den Peripherieprozessor, des Vorschaubilds an einen Schleifring der medizinischen Vorrichtung; und
Übertragen, durch den Schleifring, des Vorschaubilds an das Anzeigemodul zur Anzeige;
Durchführen, durch den Peripherieprozessor, einer Vorverarbeitungsoperation an den Abtastdaten, um verarbeitete Abtastdaten zu erzeugen;
Übertragen, durch den Peripherieprozessor, der verarbeiteten Abtastdaten an den Schleifring der medizinischen Vorrichtung, wobei die medizinische Vorrichtung eine Computertomographie- (CT) Vorrichtung beinhaltet und der Peripherieprozessor eine Edge-Computing-Vorrichtung ist, die auf einem Rotationsmodul einer Gantry der CT-Vorrichtung montiert ist;
Erhalten, durch einen Rekonstruktionsprozessor, der verarbeiteten Abtastdaten vom Schleifring der medizinischen Vorrichtung, wobei der Rekonstruktionsprozessor nicht Teil der medizinischen Vorrichtung ist;
Erzeugen, durch den Rekonstruktionsprozessor, eines Bilds in voller Qualität des Subjekts, basierend auf den verarbeiteten Abtastdaten; und
Übertragen, durch den Rekonstruktionsprozessor, des Bilds in voller Qualität an das Anzeigemodul zur Anzeige.

2. System nach Anspruch 1, wobei das Erzeugen, durch den Peripherieprozessor, eines Vorschaubilds des Subjekts, basierend auf den komprimierten Abtastdaten, Folgendes umfasst:
Erzeugen, durch den Peripherieprozessor, des Vorschaubilds des Subjekts, basierend auf den verarbeiteten Abtastdaten.

3. System nach Anspruch 2, wobei die Vorverarbeitungsoperation mindestens eine von einer Luftkorrekturverarbeitungsoperation, einer Fehlerkanalkorrekturverarbeitungsoperation, einer nichtlinearen Korrekturverarbeitungsoperation, einer Übersprechkorrekturverarbeitungsoperation, einer Kalibrierungskorrekturverarbeitungsoperation oder einer Faltungsfilterverarbeitungsoperation beinhaltet.

4. System nach Anspruch 1, wobei der mindestens eine Prozessor weiter dazu konfiguriert ist, das System anzuweisen, Operationen durchzuführen, die Folgendes beinhalten:
Übertragen, durch den Schleifring, der verarbeiteten Abtastdaten an den Rekonstruktionsprozessor; und
Erzeugen, durch den Rekonstruktionsprozessor, mindestens eines Bilds des Subjekts, basierend auf den verarbeiteten Abtastdaten.

5. System nach einem der Ansprüche 1-4, wobei das Datenerfassungsmodul ein Detektionsmodul beinhaltet.

6. Verfahren für Bildrekonstruktion, das auf einer Rechenvorrichtung implementiert ist, die mindestens einen Prozessor und mindestens eine Datenspeichervorrichtung beinhaltet, wobei das Verfahren Folgendes umfasst:
Erhalten, durch einen Peripherieprozessor, von Abtastdaten eines Subjekts von einem Datenerfassungsmodul einer medizinischen Vorrichtung, wobei das Erhalten, durch den Peripherieprozessor, von Abtastdaten des Subjekts von dem Datenerfassungsmodul der medizinischen Vorrichtung Folgendes umfasst:
Komprimieren, durch das Datenerfassungsmodul, der Abtastdaten, um komprimierte Abtastdaten zu erzeugen; und
Erhalten, durch den Peripherieprozessor, der komprimierten Abtastdaten von dem Datenerfassungsmodul;
Erzeugen, durch den Peripherieprozessor, eines Vorschaubilds des Subjekts, basierend auf den komprimierten Abtastdaten;
Übertragen, durch den Peripherieprozessor, des Vorschaubilds an ein Anzeigemodul zur Anzeige, wobei das Übertragen, durch den Peripherieprozessor, des Vorschaubilds an das Anzeigemodul zur Anzeige, Folgendes umfasst:
Übertragen, durch den Peripherieprozessor, des Vorschaubilds an den Schleifring der medizinischen Vorrichtung; und
Übertragen, durch den Schleifring, des Vorschaubilds an das Anzeigemodul zur Anzeige;
Durchführen, durch den Peripherieprozessor, einer Vorverarbeitungsoperation an den Abtastdaten, um verarbeitete Abtastdaten zu erzeugen;
Übertragen, durch den Peripherieprozessor, der verarbeiteten Abtastdaten an den Schleifring der medizinischen Vorrichtung, wobei die medizinische Vorrichtung eine Computertomographie- (CT) Vorrichtung beinhaltet und der Peripherieprozessor eine Edge-Computing-Vorrichtung ist, die auf einem Rotationsmodul einer Gantry der CT-Vorrichtung montiert ist;
Erhalten, durch einen Rekonstruktionsprozessor, der verarbeiteten Abtastdaten von einem Schleifring der medizinischen Vorrichtung, wobei der Rekonstruktionsprozessor nicht Teil der medizinischen Vorrichtung ist;
Erzeugen, durch den Rekonstruktionsprozessor, eines Bilds in voller Qualität des Subjekts, basierend auf den verarbeiteten Abtastdaten; und
Übertragen, durch den Rekonstruktionsprozessor, des Bilds in voller Qualität an das Anzeigemodul zur Anzeige.

7. Nichtflüchtiges, computerlesbares Medium, das ausführbare Anweisungen umfasst, die, wenn sie von mindestens einem Prozessor ausgeführt werden, den mindestens einen Prozessor anleiten, ein Verfahren zur Bildrekonstruktion durchzuführen, wobei das Verfahren Folgendes umfasst:
Erhalten, durch einen Peripherieprozessor, von Abtastdaten eines Subjekts von einem Datenerfassungsmodul einer medizinischen Vorrichtung, wobei das Erhalten, durch den Peripherieprozessor, von Abtastdaten des Subjekts von dem Datenerfassungsmodul der medizinischen Vorrichtung Folgendes umfasst:
Komprimieren, durch das Datenerfassungsmodul, der Abtastdaten, um komprimierte Abtastdaten zu erzeugen; und
Erhalten, durch den Peripherieprozessor, der komprimierten Abtastdaten von dem Datenerfassungsmodul;
Erzeugen, durch den Peripherieprozessor, eines Vorschaubilds des Subjekts, basierend auf den komprimierten Abtastdaten;
Übertragen, durch den Peripherieprozessor, des Vorschaubilds an ein Anzeigemodul zur Anzeige, wobei das Übertragen, durch den Peripherieprozessor, des Vorschaubilds an das Anzeigemodul zur Anzeige, Folgendes umfasst:
Übertragen, durch den Peripherieprozessor, des Vorschaubilds an den Schleifring der medizinischen Vorrichtung; und
Übertragen, durch den Schleifring, des Vorschaubilds an das Anzeigemodul zur Anzeige;
Durchführen, durch den Peripherieprozessor, einer Vorverarbeitungsoperation an den Abtastdaten, um verarbeitete Abtastdaten zu erzeugen;
Übertragen, durch den Peripherieprozessor, der verarbeiteten Abtastdaten an den Schleifring der medizinischen Vorrichtung, wobei die medizinische Vorrichtung eine Computertomographie- (CT) Vorrichtung beinhaltet und der Peripherieprozessor eine Edge-Computing-Vorrichtung ist, die auf einem Rotationsmodul einer Gantry der CT-Vorrichtung montiert ist;
Erhalten, durch einen Rekonstruktionsprozessor, der verarbeiteten Abtastdaten von einem Schleifring der medizinischen Vorrichtung, wobei der Rekonstruktionsprozessor nicht Teil der medizinischen Vorrichtung ist;
Erzeugen, durch den Rekonstruktionsprozessor, eines Bilds in voller Qualität des Subjekts, basierend auf den verarbeiteten Abtastdaten; und
Übertragen, durch den Rekonstruktionsprozessor, des Bilds in voller Qualität an das Anzeigemodul zur Anzeige.

## Revendications

1. Système de reconstruction d'images, comprenant :
au moins un dispositif de stockage incluant un ensemble d'instructions ; et
au moins un processeur configuré pour communiquer avec le au moins un dispositif de stockage, dans lequel lors de l'exécution de l'ensemble d'instructions, le au moins un processeur est configuré pour amener le système à effectuer des opérations incluant :
l'obtention, par un processeur périphérique, de données de balayage d'un sujet à partir d'un module d'acquisition de données d'un dispositif médical, dans lequel l'obtention, par le processeur périphérique, de données de balayage du sujet à partir du module d'acquisition de données du dispositif médical comprend :
la compression, par le module d'acquisition de données, des données de balayage pour générer des données de balayage compressées ; et
l'obtention, par le processeur périphérique, des données de balayage compressées du module d'acquisition de données ;
la génération, par le processeur périphérique, d'une image d'aperçu du sujet basée sur les données de balayage compressées ;
la transmission, par le processeur périphérique, de l'image d'aperçu à un module d'affichage pour affichage, dans lequel la transmission, par le processeur périphérique, de l'image d'aperçu au dispositif d'affichage pour affichage comprend :
la transmission, par le processeur périphérique, de l'image d'aperçu à une bague collectrice du dispositif médical ; et
la transmission, par la bague collectrice, de l'image d'aperçu au module d'affichage pour affichage ;
la réalisation, par le processeur périphérique, d'une opération de prétraitement sur les données de balayage pour générer des données de balayage traitées ;
la transmission, par le processeur périphérique, des données de balayage traitées à la bague collectrice du dispositif médical, dans lequel le dispositif médical inclut un dispositif de tomographie informatisée (CT), et le processeur périphérique est un dispositif de calcul de périphérie de réseau monté sur un module de rotation d'un portique du dispositif CT ;
l'obtention, par un processeur de reconstruction, des données de balayage traitées de la bague collectrice du dispositif médical, dans lequel le processeur de reconstruction ne fait pas partie du dispositif médical ;
la génération, par le processeur de reconstruction, d'une image en qualité optimale du sujet sur la base des données de balayage traitées ; et
la transmission, par le processeur de reconstruction, de l'image en qualité optimale au module d'affichage pour affichage.

2. Système selon la revendication 1, dans lequel la génération, par le processeur périphérique, d'une image d'aperçu du sujet sur la base des données de balayage comprend :
la génération, par le processeur périphérique, de l'image d'aperçu du sujet sur la base des données de balayage traitées.

3. Système selon la revendication 2, dans lequel l'opération de prétraitement inclut au moins l'une parmi une opération de traitement de correction d'air, une opération de traitement de correction de canal défectueux, une opération de traitement de correction non linéaire, une opération de traitement de correction de diaphonie, une opération de traitement de correction d'étalonnage ou une opération de traitement de filtre convolutionnel.

4. Système selon la revendication 1, dans lequel le au moins un processeur est en outre configuré pour amener le système à effectuer des opérations incluant :
la transmission, par la bague collectrice, des données de balayage traitées au processeur de reconstruction ; et
la génération, par le processeur de reconstruction, d'au moins une image du sujet sur la base des données de balayage traitées.

5. Système selon l'une quelconque des revendications 1-4, dans lequel le module d'acquisition de données inclut un module de détection.

6. Procédé de reconstruction d'image, qui est mis en œuvre sur un dispositif informatique incluant au moins un processeur et au moins un dispositif de stockage, le procédé comprenant :
l'obtention, par un processeur périphérique, de données de balayage d'un sujet à partir d'un module d'acquisition de données d'un dispositif médical, dans lequel l'obtention, par le processeur périphérique, de données de balayage du sujet à partir du module d'acquisition de données du dispositif médical comprend :
la compression, par le module d'acquisition de données, des données de balayage pour générer des données de balayage compressées ; et
l'obtention, par le processeur périphérique, des données de balayage compressées du module d'acquisition de données ;
la génération, par le processeur périphérique, d'une image d'aperçu du sujet basée sur les données de balayage compressées ;
la transmission, par le processeur périphérique, de l'image d'aperçu à un module d'affichage pour affichage, dans lequel la transmission, par le processeur périphérique, de l'image d'aperçu au module d'affichage pour affichage comprend :
la transmission, par le processeur périphérique, de l'image d'aperçu à la bague collectrice du dispositif médical ; et
la transmission, par la bague collectrice, de l'image d'aperçu au module d'affichage pour affichage ;
la réalisation, par le processeur périphérique, d'une opération de prétraitement sur les données de balayage pour générer des données de balayage traitées ;
la transmission, par le processeur périphérique, des données de balayage traitées à la bague collectrice du dispositif médical, dans lequel le dispositif médical inclut un dispositif de tomographie informatisée (CT), et le processeur périphérique est un dispositif de calcul de périphérie de réseau monté sur un module de rotation d'un portique du dispositif CT ;
l'obtention, par un processeur de reconstruction, des données de balayage traitées à partir d'une bague collectrice du dispositif médical, dans lequel le processeur de reconstruction ne fait pas partie du dispositif médical ;
la génération, par le processeur de reconstruction, d'une image en qualité optimale du sujet sur la base des données de balayage traitées ; et
la transmission, par le processeur de reconstruction, de l'image en qualité optimale au module d'affichage pour affichage.

7. Support non transitoire lisible par ordinateur, comprenant des instructions exécutables qui, lorsqu'elles sont exécutées par au moins un processeur, amènent le au moins un processeur à mettre en œuvre un procédé de reconstruction d'image, le procédé comprenant :
l'obtention, par un processeur périphérique, de données de balayage d'un sujet à partir d'un module d'acquisition de données d'un dispositif médical, dans lequel l'obtention, par le processeur périphérique, de données de balayage du sujet à partir du module d'acquisition de données du dispositif médical comprend :
la compression, par le module d'acquisition de données, des données de balayage pour générer des données de balayage compressées ; et
l'obtention, par le processeur périphérique, des données de balayage compressées du module d'acquisition de données ;
la génération, par le processeur périphérique, d'une image d'aperçu du sujet basée sur les données de balayage compressées ;
la transmission, par le processeur périphérique, de l'image d'aperçu à un module d'affichage pour affichage, dans lequel la transmission, par le processeur périphérique, de l'image d'aperçu au module d'affichage pour affichage comprend :
la transmission, par le processeur périphérique, de l'image d'aperçu à la bague collectrice du dispositif médical ; et
la transmission, par la bague collectrice, de l'image d'aperçu au module d'affichage pour affichage ;
la réalisation, par le processeur périphérique, d'une opération de prétraitement sur les données de balayage pour générer des données de balayage traitées ;
la transmission, par le processeur périphérique, des données de balayage traitées à la bague collectrice du dispositif médical, dans lequel le dispositif médical inclut un dispositif de tomographie informatisée (CT), et le processeur périphérique est un dispositif de calcul de périphérie de réseau monté sur un module de rotation d'un portique du dispositif CT ;
l'obtention, par un processeur de reconstruction, des données de balayage traitées à partir d'une bague collectrice du dispositif médical, dans lequel le processeur de reconstruction ne fait pas partie du dispositif médical ;
la génération, par le processeur de reconstruction, d'une image en qualité optimale du sujet sur la base des données de balayage traitées ; et
la transmission, par le processeur de reconstruction, de l'image en qualité optimale au module d'affichage pour affichage.
